# EUROPEAN PATENT SPECIFICATION

(11) **EP 1 743 666 B1**
(45) Date of publication and mention of the grant of the patent: **27.05.2009**
(21) Application number: 05106504.3
(22) Date of filing: 15.07.2005
(51) Int. Cl.: A61M 5/20

(54) **Injector with automatic needle insertion, injection and needle retraction**
Injektor mit automatischem Einstechen der Nadel, Injektion und Retraktion der Nadel
Injecteur avec insertion de l'aiguille, injection et retraction de l'aiguille automatiques

(43) Date of publication of application: 17.01.2007
(73) Proprietor: SHL Group AB, 131 28 Nacka Strand (SE)
(72) Inventor: Guillermo, Carlos, CA-93402, Los Osos (US)

(56) References cited:
- EP-A- 0 824 922
- WO-A-20/05011780
- FR-A- 2 519 866
- US-A1- 2004 039 336
- US-A1- 2005 016 528

## Description

### TECHNICAL FIELD

The present invention relates to an injector and in particular an injector having several automatic functions.

### BACKGROUND OF THE INVENTION

There are many injectors on the market where the aim is to have high degrees of functionality and automatic features, such as in connection with penetration, injection, setting of dose, priming and covering of the needle after use. At the same time there is a demand on robustness, repeatability and reliability regarding the function, which sometimes might be difficult to meet when dealing with complex multi functions involving many interacting components. When there further are demands on low production costs, especially for devices that are to be used only once, the picture becomes even more complex.

There are in the patent literature numerous solutions to injection devices, the bulk of which never enter the market due to that they do not meet the demands in one way or the other. There is therefore a continuous search for solutions that provide the desired functions that at the same time fulfill the functional and/or economical demands.

Manny devices having multi-functions that work in sequence, such as for example penetration, followed by injection, followed by withdrawal, have a subsequent sequence triggered at the end of a previous sequence, for example when the needle has reached full penetration depth, the injection sequence is triggered.

GB-A-728248 describes an automatic injection device comprises power means which thrusts forward on an ampoule and needle carrier causing projection of the needle into the skin of a patient. Thereafter, the power means is automatically decoupled from the needle carrier and acts on a plunger, which squeezes the contents of the ampoule through the needle. At the end, the power means is automatically decoupled from the plunger, leaving the ampoule and the needle carrier to be retracted by a return spring.

WO-A2-05011780 relates to an injection device according to the preamble of claim 1. It comprises a syringe body, a plunger with a plunger rod and an injection carriage for displacing the syringe body and the plunger. Said injection device comprises at least one actuating element that co-operates with components, which withdraw the syringe body from the injection site once the injection has been completed. A single, targeted, linear displacement inserts the needle to a defined depth, injects the medicament and, once the injection has been completed, produces the return stroke, which allows the needle to be withdrawn into the housing and thus from the injection site. Moreover, the injection device is equipped with additional components, which produce a delay between the completion of the injection and the start of the return stroke.

US-A-2005/016528 discloses a breath-operated inhaler and other medical distribution products as automatically or semi-automatically that perform different functions such as injecting the needle into the patient, delivering the medicament from the syringe and retracting the needle or ejecting a needle protector. They are provided with some kind of actuating means as springs or the like, locking means capable of holding the actuating means in an energised state, and activating means operationally attached to the actuating means and capable of releasing the locking means when the patient is to receive a dose of medicament. These actuating means could be purely manually operated, such as a button, a lever or a handle arranged on the outer surface of the device. The patient then presses or moves the activating means in order to release the locking means. The needle penetration is done by pushing the syringe forward in the housing of the injector, then this triggers the emptying of the syringe and when the syringe is emptied this triggers a needle retraction or a needle protection to be pushed forward. There could thus be a series of components or transmissions acting on a sequence.

The drawback with these solutions, especially when several components are interacting with each other in order to perform the sequences and the triggering of them, is that the tolerance and functional demands are increased in order to ensure that the subsequent sequence is really triggered at the end of the previous sequence. Should there be a tolerance error or a mismatch between two interacting components for triggering a subsequent sequence, the previous sequence will come to an end without triggering the next sequence. This may lead to a fatal situation if for example the injection of medicament is not performed.

In view of cost efficiency, high tolerance demands in order to achieve the desired chain of functions, are not advantageous, in particular when mass-producing injection devices.

### BRIEF DESCRIPTION OF THE INVENTION

The aim of the present invention is to provide an injector that fulfils the demands that are put on such devices both regarding functionality, reliability and low cost.

The invention is defined by the features of claim 1. Further advantageous features are subject of the dependent claims.

The present invention uses a method of operating an injection device, which device comprises a medicament cartridge and a needle attached to said cartridge, means for automatic penetration of needle, injection of medicament and withdrawal of needle, comprising the steps of initiating a penetration sequence, followed by an injection sequence and followed by a withdrawal sequence, wherein a previous sequence triggers a subsequent sequence, and wherein the subsequent sequence is triggered before the previous sequence has ended.

The advantage of the present invention is for one that a subsequent sequence of operation of the injector is not triggered at the end of a previous sequence. This provides an increased reliability regarding ensuring that a subsequent sequence is triggered by a previous sequence, without increasing the tolerance and functional demands on the interacting components.

The triggering of a subsequent sequence may preferably by in an end range of movement of components performing the previous sequence.

The solution provides a cost effective multi-function device comprising a safe and reliable chain of sequences, thus ensuring that the user receives a proper dose of medicament each time the device is used.

These and other features and advantages with the present invention will become apparent from the following detailed description and the drawings.

### BRIEF DESCRIPTION OF THE DRAWINGS

In the following detailed description, reference will be made to the drawings, of which
Fig. 1 is a longitudinal cross-section of an embodiment of the present invention,

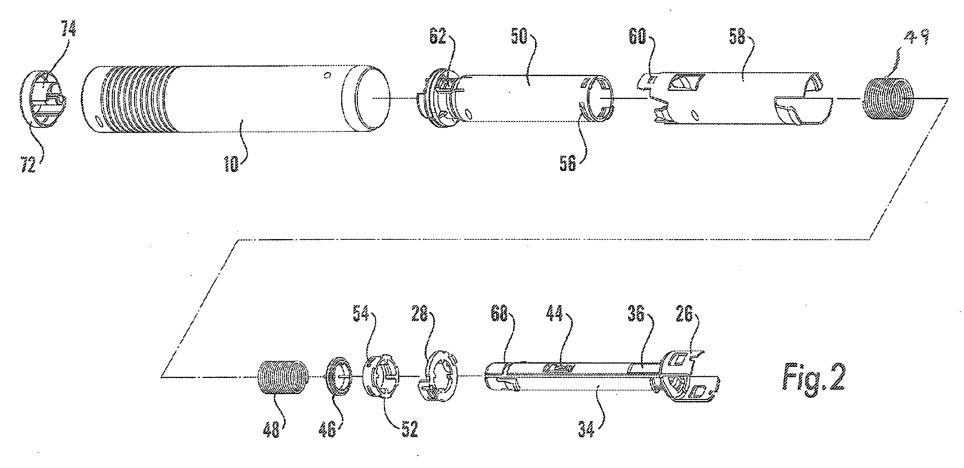

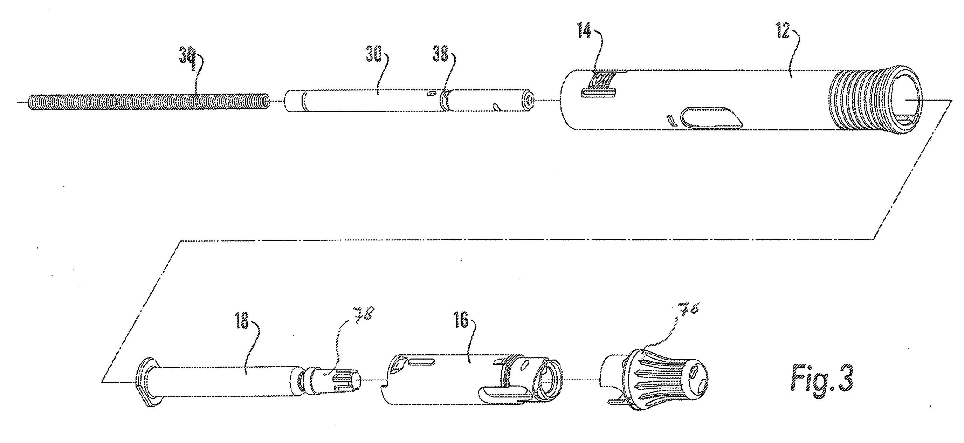

Figs. 2-3 are exploded views of the injector of Fig. 1,
Figs. 4-7b show different functional steps of the device of Fig. 1.

### DETAILED DESCRIPTION OF THE INVENTION

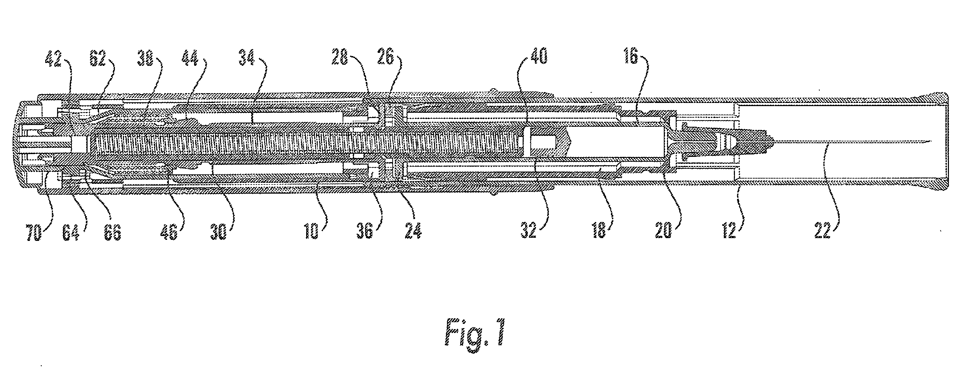

The device according to the figures comprises a generally tubular outer housing 10. In the front end of the housing, to the right in Fig. 1, a generally tubular needle shield 12 is arranged slidably in the outer housing. When in the non-extended position the needle shield is held in place by protrusions 14 on the outer surface co-operating with ledges (not shown) on the inner surface of the housing. Inside the needle shield in the front area of the device a syringe carrier 16 is arranged. Inside the syringe carrier a syringe 18, containing medicament, is arranged. The syringe is prevented from moving forward by a circumferential ledge 20. The front end of the syringe is arranged with attachment means for attaching a needle 22 to the syringe. The rear end of the syringe is seated with its flange 24 in a holder 26. Adjacent the holder an injection release ring 28 is arranged, which will be described in more detail below. A plunger 30 extends into the syringe with one end adjacent a stopper 32. The rear end of the plunger is surrounded by an activation housing 34 which is snap-fitted to the syringe holder 26. The activation housing is arranged with flexible tongues 36, where each tongue is arranged with inwardly directed ledges. In the initial state, these ledges are positioned in a circumferential groove 38 on the plunger 30. The tongues and ledges are held in this position by the injection release ring 28. Inside the plunger an injection spring 39 is arranged compressed between a front wall 40 of the plunger 30 and a wall part 42 of the activation housing 34. The activation housing is further arranged with outwardly extending protrusions arranged on flexible arms 44. Abutting the protrusions is a retraction release ring 46, which will be described closer below. A spring 48, hereafter named penetration spring, is arranged between the retraction release ring 46 and a penetration sleeve 50. At the front end of the penetration sleeve, a retraction spring retainer 52 is snap fitted with the penetration sleeve 50 by outwardly directed protrusions 54 having a straight part and a ramped part, extending into recesses 56 of the sleeve. Outside the penetration sleeve a lockout sleeve 58 is arranged. At the rear part of the lockout sleeve 58 recesses 60 are arranged adjacent flexible arms 62 of the penetration sleeve, which arms are arranged with outwardly extending protrusions 64 as well as inwardly extending ledges 66. In the initial position these ledges are in contact with a wall of a circumferential groove 68 on the activation housing 34. The upper part of the arms is further arranged with inclined surfaces 70. At the upper end of the housing an activation button 72 is slidably arranged, having inwardly extending parts 74, which are arranged with inclined surfaces facing the inclined surfaces 70 of the arms 62.

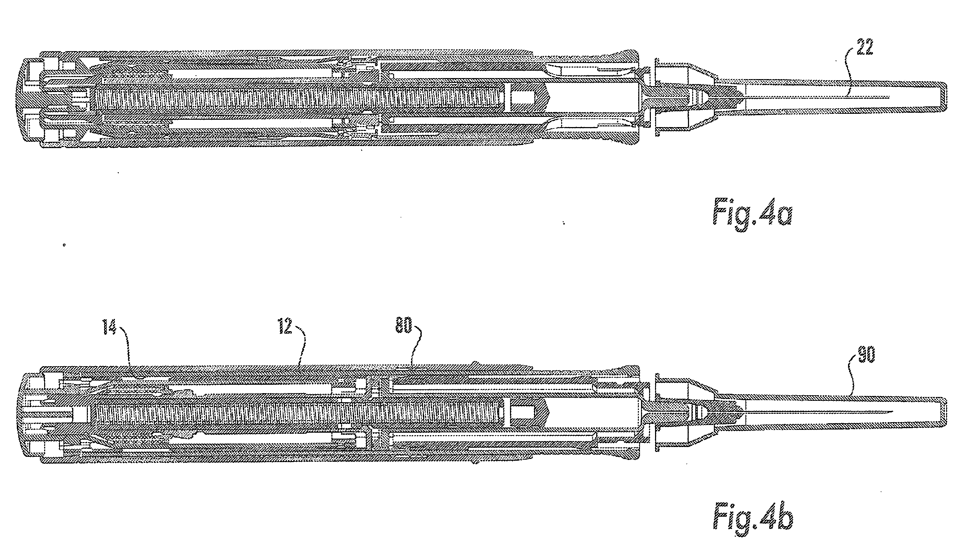

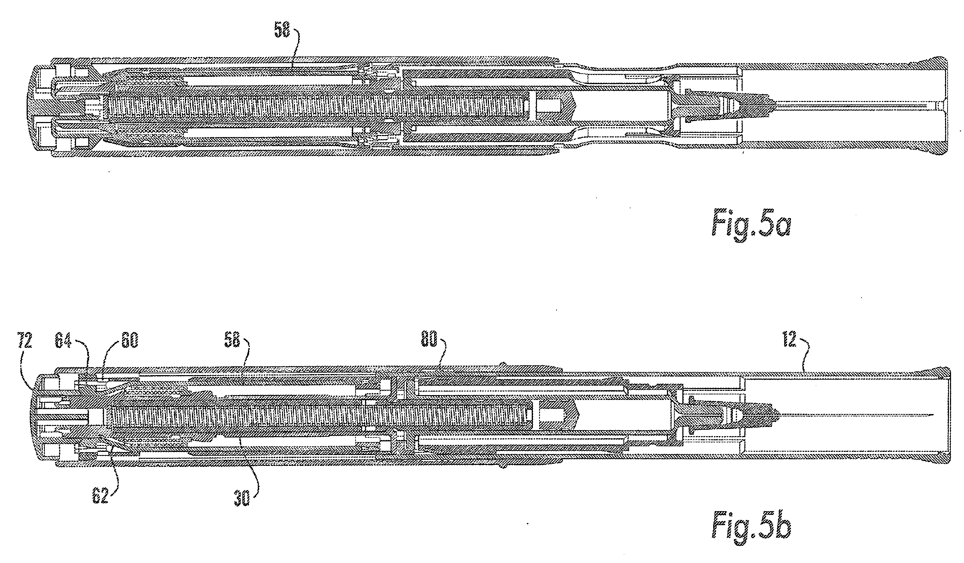

The device is intended to function as follows. When in the initial position the needle shield 12 is pushed inside the housing 10 and held in place by the protrusions 14 acting against the ledge of the housing, Fig 4b. The device is delivered with a protective cap 76 inserted into the front end of the needle shield surrounding the front end of the syringe 18 with its syringe cap 78. The protective cap is removed, whereby the syringe cap is also removed, and a needle 22 is attached to the syringe. The needle shield 12 is then pushed manually forward until the protrusions 14 of the needle shield enter a recess 80 on the inner surface of the housing 10, Fig. 5b. The protrusions have such a configuration that they are able to slide over the ledge when the needle shield is extended but prevent a pushing in of the needle shield when they have entered the recess. The forward movement of the needle shield pushes a protective needle cap 90 off the needle.

The device is now ready to use. The user places the end of the needle shield 12 against the injection site and presses the push button 72. The pressing of the needle shield 12 causes it to move a short distance inwards until the protrusions 14 of the needle shield abut the upper wall of the recess 80. This movement causes the lockout sleeve 58 to be moved the same short distance since the upper end of the needle shield 12 in the extended position is in contact with the lower end of the lockout sleeve 58.

If the device is withdrawn from the injection site the lockout sleeve and the needle shield are moved back to initial position.

The movement of the lockout sleeve causes its recesses 60 to be positioned outside of the outwardly extending protrusions 64 of the arms 62 of the penetration sleeve 58, which enables the button 72 to be depressed whereby the inclined surfaces of the inwardly extending parts act on the inclined surfaces 70 of the arms 62, causing them to move radially outwards. This is not possible when the lockout sleeve 58 has not been moved since the protrusions of the arms then abut the inner surface of the lockout sleeve.

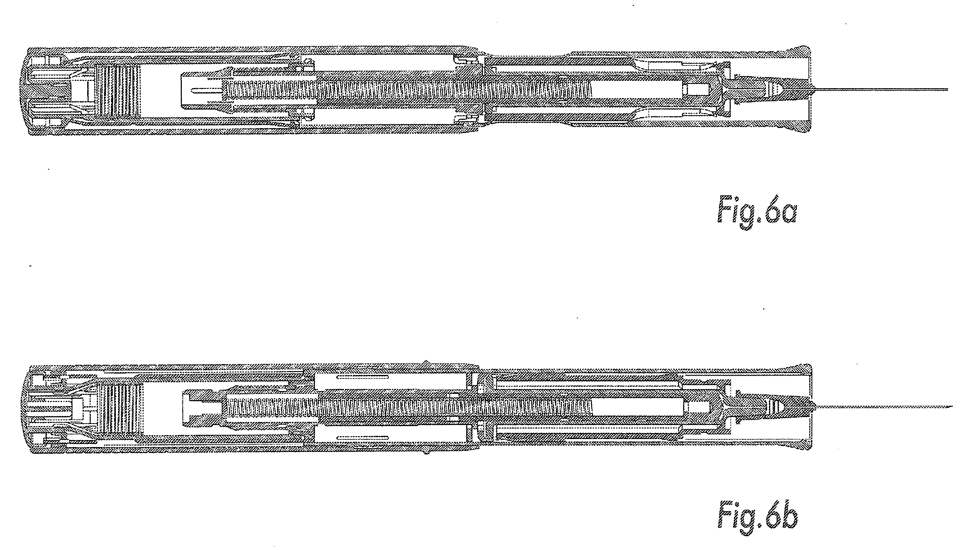

When the arms move radially outwards the inwardly directed ledges 66 of the arms 62 are moved out of contact with the circumferential groove 68 of the activation housing 34 which then is moved forward by the penetration spring 48 acting on the retraction release ring 46 which is held in place relative the activation housing 34 by the protrusions 44. Thus both the activation housing 34, the plunger 30 arranged inside the activation housing, the syringe carrier 16 connected to the activation housing, the injection release ring 28 and the syringe 18 are moved forward causing a penetration of the needle into the injection site, Fig. 6.

At a certain depth the injection release ring 28 is stopped by the engagement of protrusions on its flexible arms into slots on the shield, which frees the flexible tongues 36 because they pass the ring due to the continued movement of the activation housing 34. The freeing of the tongues cause them to flex outwards radially, whereby the inwardly directed ledges are moved out of contact with the groove 38 on the plunger 30. The movement of the activation housing 34, and thus the penetration, is stopped when the retraction release ring 46 contacts the retraction spring retainer 52.

However the plunger is now free to move by the force of the injection spring 39, whereby it pushes on the stopper 32 and an injection is performed. The movement of the plunger in relation to the activation housing 34 causes the upper end of the plunger to pass the protrusions 44 of the activation housing, whereby they are capable of collapsing inwards.

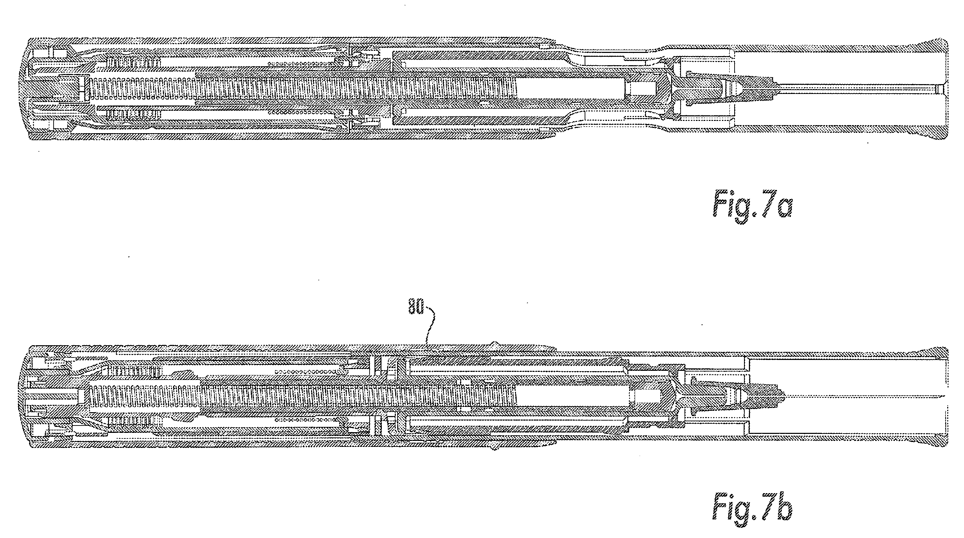

The collapsing causes the retraction release ring to pass the protrusions 44 and to be pushed downwards by the retraction spring 49. This causes the syringe carrier 16 to be pulled into the housing via the retraction spring retainer at near end of injection stroke, and thus the needle to be retracted, Fig. 7. The injection is now completed and the needle is protected inside the housing.

It is to be understood that the embodiment described above and shown in the drawings is to be regarded only as a non-limiting example of the invention and that it may be modified within the scope of the patent claims.

## Claims

1. Injection device comprising a housing (10), a needle shield (12), a medicament cartridge (18), a needle (22) attached to said cartridge, means (34, 48, 50, 58, 72) for an automatic needle penetration, means (28, 30, 34, 39) for an automatic medicament injection and means (34, 46, 49) for an automatic needle withdrawal,
**characterised in that**
said penetration, injection and withdrawal means comprises a joint single-component activation housing (34) having penetration trigger means (68) capable of initiating a penetration sequence, injection trigger means (36) capable of initiating an injection sequence and withdrawal trigger means (44) capable of initiating a needle withdrawal; wherein said trigger means are arranged to said activation housing and designed such that a subsequent sequence is triggered before the previous sequence has ended.

2. Injection device according to claim 1, **characterised in that** the needle shield (12) is arranged inside the housing (10) for allowing the attachment of a needle to the cartridge.

3. Injection device according to claim 2, **characterised in that** the needle shield (12) comprises protrusions (14) capable of entering a recess (80) on the inner surface of the housing (10) when said needle shield is manually pushed forward for covering said needle.

4. Injection device according to any of the preceding claims, **characterised in that** the automatic needle penetration means comprises an activation button (72), a penetration spring (48), a penetration sleeve (50) and a lockout sleeve (58) wherein said lockout sleeve is arranged and designed to allow said penetration sleeve to interact with said penetration trigger means (68) for initiating the penetration sequence after said needle shield is pressed against an injection site and said push button is manually operated.

5. Injection device according to any of the preceding claims, **characterised in that** the medicament injection means comprises an injection release ring (28), a plunger (30) and an injection spring (39), wherein said injection release ring is arranged and designed to interact with said injection trigger means (36) for initiating the injection sequence.

6. Injection device according to any of the preceding claims, **characterised in that** the withdrawal means comprises a retraction release ring (46) and a retraction spring (49), wherein said retraction release ring is arranged and designed to interact with said withdrawal trigger means (44) for initiating the withdrawal sequence.

7. Injection device according to any of the preceding claims, **characterised in that** said penetration trigger means (68) is arranged and designed as a circumferential groove.

8. Injection device according to any of the preceding claims, **characterised in that** said injection trigger means (36) is arranged and designed as flexible tongues having inwardly directed ledges.

9. Injection device according to any of the preceding claims, **characterised in that** said withdrawal trigger means (44) is arranged and designed as flexible arms having inwardly directed ledges having outwardly extending protrusions.

## Patentansprüche

1. Injektionsvorrichtung mit einem Gehäuse (10), einem Nadelschutz (12), einer Arzneimittelpatrone (18), einer an der Patrone angebrachten Nadel (22), einem Mittel (34, 48, 50, 58, 72) für einen automatischen Nadeleinstich, einem Mittel (28, 30, 34, 39) für eine automatische Arzneimittelinjektion und einem Mittel (34, 46, 49) für einen automatischen Nadelrückzug,
**dadurch gekennzeichnet, dass**
das Einstich-, das Injektions- und das Rückzugmittel ein gemeinsames Einkomponenten-Betätigungsgehäuse (34) umfassen, das ein Einstichauslösemittel (68), das eine Einstichsequenz einleiten kann, ein Injektionsauslösemittel (36), das eine Injektionssequenz einleiten kann, und ein Rückzugauslösemittel (44), das einen Nadelrückzug einleiten kann, aufweist, wobei alle Auslösemittel am Betätigungsgehäuse angeordnet und so ausgelegt sind, dass eine nachfolgende Sequenz ausgelöst wird, bevor die vorherige Sequenz beendet ist.

2. Injektionsvorrichtung nach Anspruch 1, **dadurch gekennzeichnet, dass** der Nadelschutz (12) im Gehäuse (10) angeordnet ist, damit eine Nadel an der Patrone angebracht werden kann.

3. Injektionsvorrichtung nach Anspruch 2, **dadurch gekennzeichnet, dass** der Nadelschutz (12) Vorsprünge (14) umfasst, die in eine Ausnehmung (80) an der Innenfläche des Gehäuses (10) eintreten können, wenn der Nadelschutz von Hand zum Abdecken der Nadel vorgeschoben wird.

4. Injektionsvorrichtung nach einem der vorhergehenden Ansprüche, **dadurch gekennzeichnet, dass** das Mittel für einen automatischen Nadeleinstich eine Betätigungstaste (72), eine Einstichfeder (48), eine Einstichhülse (50) und eine Verriegelungshülse (58) umfasst, wobei die Verriegelungshülse so angeordnet und ausgelegt ist, dass die Einstichhülse mit dem Einstichauslösemittel (68) zusammenwirken kann, um die Einstichsequenz einzuleiten, nachdem der Nadelschutz gegen eine Injektionsstelle gedrückt und die Drucktaste von Hand betätigt worden ist.

5. Injektionsvorrichtung nach einem der vorhergehenden Ansprüche, **dadurch gekennzeichnet, dass** das Arzneimittelinjektionsmittel einen Injektionsfreigabering (28), einen Stößel (30) und eine Injektionsfeder (39) umfasst, wobei der Injektionsfreigabering so angeordnet und ausgelegt ist, dass er mit dem Injektionsauslösemittel (36) zusammenwirkt, um die Injektionssequenz einzuleiten.

6. Injektionsvorrichtung nach einem der vorhergehenden Ansprüche, **dadurch gekennzeichnet, dass** das Rückzugmittel einen Rückzugfreigabering (46) und eine Rückzugfeder (49) umfasst, wobei der Rückzugfreigabering so angeordnet und ausgelegt ist, dass er mit dem Rückzugauslösemittel (44) zusammenwirkt, um die Rückzugsequenz einzuleiten.

7. Injektionsvorrichtung nach einem der vorhergehenden Ansprüche, **dadurch gekennzeichnet, dass** das Einstichauslösemittel (68) als eine Umfangsnut angeordnet und ausgelegt ist.

8. Injektionsvorrichtung nach einem der vorhergehenden Ansprüche, **dadurch gekennzeichnet, dass** das Injektionsauslösemittel (36) als flexible Zungen mit nach innen weisenden Absätzen angeordnet und ausgelegt ist.

9. Injektionsvorrichtung nach einem der vorhergehenden Ansprüche **dadurch gekennzeichnet, dass** das Rückzugauslösemittel (44) als flexible Arme mit nach innen weisenden Absätzen mit sich nach außen erstreckenden Vorsprüngen angeordnet und ausgelegt ist.

## Revendications

1. Dispositif d'injection comprenant un boîtier (10), un protège-aiguille (12), une cartouche de médicament (18), une aiguille (22) attachée à ladite cartouche, des moyens (34, 48, 50, 58, 72) pour une pénétration automatique de l'aiguille, des moyens (28, 30, 34, 39) pour une injection automatique de médicament et des moyens (34, 46, 49) pour un retrait automatique de l'aiguille,
**caractérisé en ce que**
lesdits moyens de pénétration, d'injection et de retrait comprennent un boîtier d'activation commun (34) à un seul composant ayant des moyens de déclenchement de pénétration (68) capables d'amorcer une séquence de pénétration et des moyens de déclenchement de retrait (44) capables d'amorcer le retrait d'une aiguille ; tous lesdits moyens de déclenchement étant agencés par rapport audit boîtier d'activation et étant conçus de telle sorte qu'une séquence subséquente soit déclenchée avant la fin de la séquence précédente.

2. Dispositif d'injection selon la revendication 1, **caractérisé en ce que** le protège-aiguille (12) est arrangé à l'intérieur du boîtier (10) pour permettre d'attacher une aiguille à la cartouche.

3. Dispositif d'injection selon la revendication 2, **caractérisé en ce que** le protège-aiguille (12) comprend des saillies (14) capables d'entrer dans un enfoncement (80) de la surface interne du boîtier (10) lorsque ledit protège-aiguille est poussé manuellement vers l'avant pour recouvrir ladite aiguille.

4. Dispositif d'injection selon l'une quelconque des revendications précédentes, **caractérisé en ce que** les moyens de pénétration automatique de l'aiguille comprennent un bouton d'activation (72), un ressort de pénétration (48), un manchon de pénétration (50) et un manchon de verrouillage (58), ledit manchon de verrouillage étant agencé et conçu pour permettre audit manchon de pénétration d'interagir avec lesdits moyens de déclenchement de pénétration (68) pour amorcer la séquence de pénétration après que ledit protège-aiguille a été pressé contre un site d'injection et que ledit bouton poussoir a été actionné manuellement.

5. Dispositif d'injection selon l'une quelconque des revendications précédentes, **caractérisé en ce que** les moyens d'injection de médicament comprennent une bague de libération d'injection (28), un plongeur (30) et un ressort d'injection (39), ladite bague de libération d'injection étant agencée et conçue pour interagir avec ledit moyen de déclenchement d'injection (36) pour amorcer la séquence d'injection.

6. Dispositif d'injection selon l'une quelconque des revendications précédentes, **caractérisé en ce que** les moyens de retrait comprennent une bague de libération de retraction (46) et un ressort de rétraction (49), ladite bague de libération de rétraction étant agencée et conçue pour interagir avec lesdits moyens de déclenchement de retrait (44) pour amorcer la séquence de retrait.

7. Dispositif d'injection selon l'une quelconque des revendications précédentes, **caractérisé en ce que** lesdits moyens de déclenchement de pénétration (68) sont agencés et conçus sous forme de gorge circonférentielle.

8. Dispositif d'injection selon l'une quelconque des revendications précédentes, **caractérisé en ce que** lesdits moyens de déclenchement d'injection (36) sont agencés et conçus sous forme de langues flexibles ayant des rebords dirigés vers l'intérieur.

9. Dispositif d'injection selon l'une quelconque des revendications précédentes, **caractérisé en ce que** lesdits moyens de déclenchement de retrait (44) sont agencés et conçus sous forme de bras flexibles ayant des rebords dirigés vers l'intérieur ayant des saillies s'étendant vers l'extérieur.
